# EUROPEAN PATENT APPLICATION

(11) **EP 3 437 665 A1**
(43) Date of publication of application: **06.02.2019**
(21) Application number: 17774011.5
(22) Date of filing: 28.02.2017
(51) Int. Cl.: A61L 31/08

(54) **LIQUID MATERIAL FOR MEDICAL DEVICE COATING AND MEDICAL DEVICE HAVING SLIDING COATING FILM**

(30) Priority: 31.03.2016 JP 2016072296
(71) Applicant: Terumo Kabushiki Kaisha, Tokyo 151-0072 (JP)
(72) Inventor: ABE, Yoshihiko, Ashigarakami-gun Kanagawa 259-0151 (JP); KIMINAMI, Hideaki, Ashigarakami-gun Kanagawa 259-0151 (JP); UEDA, Tsutomu, Ashigarakami-gun Kanagawa 259-0151 (JP)
(74) Representative: Casalonga
(86) International application number: PCT/JP2017/007833
(87) International publication number: WO 2017/169466

(57) **Abstract**

The liquid material for medical device coating of the present invention contains first polysiloxane having a silanol group only at one end thereof, second polysiloxane having the silanol group at both ends thereof, and polyfunctional silane. The medical device (gasket 1) of the present invention has a slidable coating layer 3 formed of the above-described coating liquid material.

## Description

### TECHNICAL FIELD

The present invention relates to a liquid material for medical device coating capable of imparting stable slidability and a medical device having slidable coating layer and the stable slidability.

### BACKGROUND ART

A prefilled syringe in which a liquid medicine is filled in advance has been conventionally used to prevent the use of a wrong medical agent, prevent hospital infection, reduce waste, and increase efficiency in hospital service. Syringes are constructed of a barrel, a gasket slidable inside the syringe, and a plunger for operating the movement of the gasket. To enhance the sliding performance of the gasket for the syringe and obtain a high degree of flow accuracy without generating a large irregularity in the discharge of the liquid medicine from the syringe, silicone oil or the like is applied as a lubricant to a sliding portion of the outer surface of the gasket or the inner surface of the syringe. But it is known that in dependence on the kind of the liquid medicine, an interaction occurs between the liquid medicine and the lubricant such as the silicone oil. When the liquid medicines are stored for a long time after the liquid medicine is filled in the syringe, some kinds of medical agent are modified by the interaction between the medical agents and the silicone oil. Thus, it is difficult to use some kinds of medical agents to fill them in syringes in advance. The prefilled syringe for storing the liquid medicine for a long term with the liquid medicine being filled therein is demanded to keep the liquid medicine stable for a long term and eliminate the need for the use of the lubricant. To solve the above-described problem, as disclosed in a patent document 1 (Japanese Patent Application Laid-Open Publication No. 62-32970), a patent document 2 (Japanese Patent Application Laid-Open Publication No. 2002-089717), and a patent document 3 (U.S. Patent No. 7111848), prefilled syringes were proposed in which the surface of the gasket is covered with the fluorine resin which is a material having a lower friction coefficient than the material of the gasket body to eliminate the need for the use of the lubricant.

The present applicant proposed the gasket having the coating layer composed of the fluorine resin, the silicon resin, and the urethane resin, as disclosed in a patent document 4 (Japanese Patent Application Laid-Open Publication No. 2004-321614); the gasket having the coating layer composed of the layer made of the composition containing the slidability-imparting component and the flexibility-imparting component and of the layer consisting of the fine solid particles held by the layer to form the rough surface on the gasket, as disclosed in a patent document 5 (Japanese Patent Application Laid-Open Publication No. 2006-167110) and a patent document 6 (Japanese Patent Application Laid-Open Publication No. 2008-287). As also disclosed in a patent document 7 (WO Publication No. 2009-084646), the present applicant devised the composition containing the slidability-imparting component, the flexibility-imparting component, and the adhesive property and proposed the gasket having the coating layer consisting of the composition and not containing the fine solid particles.

### PRIOR ART DOCUMENT

### PATENT DOCUMENT

Patent document 1: Japanese Patent Application Publication Laid-Open No. 62-32970
Patent document 2: Japanese Patent Application Laid-Open Publication No. 2002-089717
Patent document 3: US Patent Publication No. 7111848
Patent document 4: Japanese Patent Application Laid-Open Publication No. 2004-321614
Patent document 5: Japanese Patent Application Laid-Open Publication No. 2006-167110
Patent document6: Japanese Patent Application Laid-Open Publication No. 2008-287 (United States Patent Application Publication No. 2007-0299402)
Patent document 7: WO Publication No. 2009-084646 (USP Patent No. 8968260, EP Patent Application Publication No. 2226088)

### SUMMARY OF THE INVENTION

### PROBLEM TO BE SOLVED BY THE INVENTION

The gaskets disclosed in the patent document 1 (Japanese Patent Application Laid-Open Publication No. 62-32970), the patent document 2 (Japanese Patent Application Laid-Open Publication No. 2002-089717), and the patent document 3 (U.S. Patent Publication No. 7111848) are expected to be effective in dependence on a use condition. But a formulation for a prefilled syringe is demanded to discharge the liquid medicine from the syringe under a high pressure and have the performance of stably discharging the liquid medicine therefrom little by little with a very high accuracy for a long time by using a syringe pump or the like. Thus, liquid-tightness and slidability which are fundamental performance demanded for the syringe are still in a trade-off relationship.

As described above, there is a demand for the development of a syringe which has the liquid-tightness and slidability favorably compatible with each other and eliminates the need for the use of the lubricant. In administration of the liquid medicine by using the syringe pump, when the liquid medicine is discharged from the syringe in a condition where the flow rate is so low (for example, in the case of a syringe having a diameter of about 24mm, the movement speed of the liquid medicine is about 2mm/hour when the liquid medicine is discharged from the syringe at a speed of 1mL/hour) that the flow of the liquid medicine is invisible, an unstable discharge state called pulsation is liable to occur. Thus, there is a fear that accurate administration of the liquid medicine is prevented.

The gaskets disclosed in the patent document 4 (Japanese Patent Application Laid-Open Publication No. 2004-321614), the patent document 5 (Japanese Patent Publication Laid-Open No. 2006-167110), and the patent document 6 (Japanese Patent Application Laid-Open No. 2008-287) were proposed to balance liquid-tight property with slidability. These gaskets are liquid-tight and have stable sliding performance although a lubricant is not applied to the sliding surface thereof. But the former has a problem in terms of production and cost in that the kinds of the materials forming the coating layer range widely. The latter has also a problem that the solid fine particles held by the coating layer separate therefrom and thus, the insoluble fine particles are generated in the liquid medicine.

The gasket disclosed in the patent document 7 (Japanese Patent Application Laid-Open Publication No. 2007-339649) solves the above-described problems. In prefilled syringes in which biotechnology-based medical agents are filled, lubricants such as silicone oil are not used to prevent medical agents from coagulating due to the use of silicone oil. Thus, the non-use of the silicone oil is very effective. Some kinds of biotechnology-based medical agents have a high viscosity. Thus, in a recent trend, there is a demand for the development of a medical device-coating material and a medical device having slidable coating layer which eliminate the need for imparting a lubricant to a sliding surface and has a low sliding resistance value and a stable slidability.

The present invention is intended to solve the above-described problems. Thus, it is an object of the present invention to provide a liquid material for medical device coating and a medical device having slidable coating layer capable of achieving stable sliding performance at a lower sliding resistance value without imparting a lubricant to a sliding surface.

The liquid material for medical device coating of the present invention achieving the above-described object has a form as described below.

The liquid material for medical device coating for imparting slidability to a medical device contains first polysiloxane having a silanol group only at one end thereof, second polysiloxane having the silanol group at both ends thereof, and polyfunctional silane.

The medical device having slidable coating layer of the present invention achieving the above-described object has a form as described below.

The medical device having slidable coating layer contacts a medical member or is inserted into a living body when the medical device having slidable coating layer is used. The medical device having slidable coating layer has a slidable coating layer formed at a portion thereof where the medical device contacts the medical member or an inside of the living body. The slidable coating layer has a main structure containing a condensate of reactive polysiloxane having a silanol group at both ends thereof as a main component thereof and a large number of crosslinking parts formed of polyfunctional silane. In addition, The slidable coating layer contains polysiloxane having one end which is bonded to the crosslinking parts and having other end which does not have a reactive functional group and is not bonded to the main structure nor to the crosslinking parts. The polysiloxane having the one end and the other end is bonded to at least one part of the crosslinking parts.

The syringe of the present invention achieving the above-described object has a form as described below.

The syringe has a barrel, a gasket for the syringe slidably accommodated inside the barrel, and a plunger which has been mounted on the gasket or can be mounted thereon.

### BRIEF DESCRIPTION OF THE DRAWINGS

Fig. 1 is a front view of a gasket for a syringe of an embodiment of a medical device having slidable coating layer of the present invention.
Fig. 2 is a sectional view of the gasket for the syringe shown in Fig. 1.
Fig. 3 is a plan view of the gasket for the syringe shown in Fig. 1.
Fig. 4 is a bottom view of the gasket for the syringe shown in Fig. 1.
Fig. 5 is a longitudinal sectional view of a prefilled syringe of an embodiment of the medical device having slidable coating layer of the present invention.
Fig. 6 is a sectional view of a guide wire of an embodiment of the medical device having slidable coating layer of the present invention.

### MODE FOR CARRYING OUT THE INVENTION

The liquid material for medical device coating of the present invention and the medical device having slidable coating layer thereof are described below.

First of all, the liquid material for medical device coating of the present invention is described below.

The liquid material for medical device coating of the present invention contains first polysiloxane having a silanol group only at one end thereof, second polysiloxane having the silanol group at both ends thereof, and polyfunctional silane.

An aqueous solvent is used for the liquid material for medical device coating of the present invention. Water is suitable as the aqueous solvent. Alcohol, a surface active agent, and the like may be added to the aqueous solvent. It is preferable that the liquid material for medical device coating consists of an aqueous emulsion. Although the liquid material for medical device coating may be of a thermosetting type or a normal temperature-curing type, it is preferable that the liquid material for medical device coating is a thermosetting coating liquid material in view of workability thereof.

In adding the surface active agent to the aqueous solvent, an anion surface active agent is preferable as the surface active agent. Any anion surface active agent may be used. It is possible to use aliphatic monocarboxylates, polyoxyethylene alkyl ether carboxylates, N-acyl sarcosinates, N-acyl glutamates, dialkyl sulfosuccinates, alkane sulfonates, alpha olefin sulfonates, straight chain alkylbenzene sulfonates, molecular chain alkylbenzene sulfonates, naphthalene sulfonate-formaldehyde condensate, alkyl naphthalene sulfonates, N-methyl-N-acyl taurine, alkyl sulfate, polyoxyethylene alkyl ether sulfates, sulfated oil, alkyl phosphates, polyoxyethylene alkyl ether sulfates, and polyoxyethylene alkyl phenyl ether sulfates.

A nonionic surface active agent may be used. Any nonionic surface active agents may be used. It is possible to use polyoxyethylene alkyl ether, polyoxyalkylene derivatives, polyoxyethylene alkyl phenyl ether, polyoxyethylene sorbitan fatty acid ester, fatty acid alkanolamide, glycerin fatty acid ester, sorbitan fatty acid ester, polyoxyethylene alkylamine, and alkyl alkanolamide.

As the first polysiloxane having the silanol group at only one end thereof, polysiloxane having the silanol group at only one end thereof and an unreactive group (unreactive functional group) at the other end thereof is used. More specifically, as the first polysiloxane, polydimethylsiloxane having the silanol group at one end thereof and a trimethylsilyl group at the other end thereof is preferable.

The first polysiloxane (more specifically, polydimethylsiloxane) having a molecular weight of 3,000 to 50,000 is favorable. The first polysiloxane having a molecular weight of 4,000 to 30,000 is more favorable.

The first polysiloxane can be said to be reactive silicone having the silanol group at its one end. More specifically, first polysiloxane having the silanol group at one end thereof and trialkylsilyl polydimethylsiloxane at the other end thereof is preferable. It is possible to exemplify first polysiloxane having the silanol group at one end thereof and trimethylsilyl polydimethylsiloxane at the other end thereof, first polysiloxane having the silanol group at one end thereof and triethylsilyl polydimethylsiloxane at the other end thereof, and the like are preferable.

Although the form of the first polysiloxane is not specifically limited, an emulsion consisting of the above-described polysiloxane dispersedly emulsified in an aqueous medium is preferable.

The second polysiloxane (more specifically, polydimethylsiloxane) having a molecular weight of 200,000 to 500,000 is favorable. The second polysiloxane having a molecular weight of 250,000 to 400,000 is more favorable.

The second polysiloxane can be said to be reactive polysiloxane having the silanol group at both ends thereof. As the second polysiloxane, polysiloxane-based silicone such as polydimethylsiloxane having the silanol group at both ends thereof is preferable. It is possible to exemplify polydimethylsiloxane having the silanol group at both ends thereof, poly diphenylsiloxane having the silanol group at both ends thereof, diphenylsiloxane-dimethylsiloxane copolymer having the silanol group at both ends thereof, and the like.

Although the form of the second polysiloxane is not specifically limited, the above-described reactive polysiloxane compounds or polysiloxane consisting of a condensate of the reactive polysiloxane compound is used by dispersing, emulsifying or dissolving these substances in an aqueous medium. As the form of the second polysiloxane, it is possible to use an emulsion formed by compositing the polysiloxane with an organic polymer.

The ratio between the content of the first polysiloxane and the second polysiloxane contained in the liquid material for medical device coating is favorably 50:50 to 5:95 and more favorably 40:60 to 10:90.

Polyfunctional silane having at least two reactive functional groups are used. It is preferable that the polyfunctional silane has three reactive functional groups. Any of the polyfunctional silane having a plurality of reactive functional groups identical to each other or partly or all different from each other is acceptable.

It is preferable that the polyfunctional silane is any one of nitrogen-containing silane, epoxy silane, and alkyl silane or the combination of not less than two kinds thereof. More specifically, as the polyfunctional silane, alkylalkoxysilane, phenylalkoxysilane, alkyl phenoxy silane, aminoalkyl alkoxysilane, and glycidoxy alkyl alkoxysilane are preferable.

It is preferable that the liquid material for medical device coating of the present invention contains the alkylalkoxysilane or the phenylalkoxysilane as first polyfunctional silane, the nitrogen-containing silane as second polyfunctional silane, or/and the glycidoxy alkyl alkoxysilane as third polyfunctional silane.

As the first polyfunctional silane, the alkylalkoxysilane, the alkyl phenoxy silane, the phenylalkoxysilane, and the like are preferable. The alkylalkoxysilane has at least one alkyl group whose carbon number is 1 to 20 and at least one alkoxy group whose carbon number is 1 to 4.

Preferable first polyfunctional silane include methyltrimethoxysilane, methyltriethoxysilane, methyltriisobutoxysilane, methyl tributoxy silane, methyl_{sec}-trioctyloxysilane, isobutyltrimethoxysilane, cyclohexyl methyl dimethoxy silane, diisopropyl dimethoxysilane, propyl trimethoxysilane, diisobutyl dimethoxysilane, n-octylmethoxysiloxane, ethyltrimethoxysilane, dimethyldimethoxysilane, octyltriethoxysilane, hexyl trimethoxysilane, hexyl triethoxysilane, octamethylcyclotetrasiloxane, methyltri(acryloyloxyethoxy)silane, octyltriethoxysilane, lauryl triethoxy silane, stearyl trimethoxysilane, stearyl triethoxtsilane, ethyltriethoxysilane, propyltriethoxysilane, butyltrimethoxysilane, butyltriethoxysilane, pentyl trimethoxysilane, pentyl triethoxysilane, heptyl trimethoxysilane, heptyl triethoxysilane, octyltrimethoxysilane, nonyl trimethoxy silane, nonyl triethoxy silane, decyl trimethoxysilane, decyl triethoxysilane, undecyl trimethoxysilane, undecyl triethoxysilane, dodecyl trimethoxysilane, dodecyl triethoxysilane, tridecyl trimethoxysilane, tridecyl triethoxysilane, tetradecyl trimethoxysilane, tetradecyl triethoxysilane, pentadecyl trimethoxysilane, pentadecyl triethoxysilane, hexadecyltrimethoxysilane, hexadecyltriethoxysilane, heptadecyl trimethoxysilane, heptadecyl triethoxysilane, octadecyl trimethoxysilane, octadecyl triethoxysilane, nonadecyl trimethoxysilane, nonadecyl triethoxysilane, eicosyl trimethoxysilane, and eicosyl triethoxysilane.

As the alkylphenoxysilane, methyltriphenoxysilane is preferable. As the phenoxyalkoxysilane, phenyltrimethoxysilane, phenyltriethoxysilane, diphenyldimethoxysilane, and diphenyldiethoxysilane are preferable.

As the first polyfunctional silane, it is possible to use methyltri(glycidyloxy)silane, trimethylchlorosilane, dimethylchlorosilane, methyltrichlorosilane, tetraethoxysilane, heptadecafluorodecyltrimethoxysilane, tridecafluoro octyl trimethoxy silane, and tetra propoxy silane.

The mixing amount of the polyfunctional silane to be contained in the liquid material for medical device coating of the present invention is favorably 0.01 to 10wt% and more favorably 0.1 to 5wt% for the first and second polysiloxanes. When the mixing amount of the polyfunctional silane is less than 0.1wt%, it is difficult to allow the coating liquid material to be sufficiently stable. When the mixing amount thereof exceeds 10wt%, the coating layer has an insufficient adhesion to the base material, which is not preferable.

As the nitrogen-containing silane of the second polyfunctional silane, alkoxysilane having a ureido group (-NH-CO-NH2) and alkoxysilane having an uraren group (-NH-CO-NH-) are exemplified. As the alkoxysilane having the ureido group (-NH-CO-NH₂) and the alkoxysilane having the uraren group (-NH-CO-NH-), γ-ureidopropyltriethoxysilane, γ-ureidopropyldiethoxymethylsilane, methylurarenpropyldimethoxymethylsilane, 3- [(2-ureidoethyl)uriel]propyl trimethoxysilane, O=C[NHCH₂CH₂CH₂Si(OC₂H₅)₃]₂ are listed.

In view of the inclusion of the nitrogen-containing silane in an aqueous emulsion, the γ-ureidopropyltriethoxysilane is preferable because it is watersoluble and thus water-dispersible and in addition easily commercially available.

As other polyfunctional silanes, a reaction product of alkoxysilane having amino group and dicarboxylic acid anhydride is preferable. It is possible to obtain the reaction product by mixing the alkoxysilane having the amino group and the dicarboxylic anhydride with each other at a mixing ratio of the amino group to the carboxylic acid set to 0.5 to 2 and favorably 0.8 to 1.2 in a mole rate and making a reaction therebetween in a solvent for several hours to more than ten hours at a room temperature to 90 degrees C. As solvents to be used, alcohols such as methanol, ethanol, and isopropanol; and ketones such as acetone and methyl ethyl ketone are listed. It is preferable to make the reaction between the above-described two substances while the solvent is refluxing.

As the alkoxysilane having the amino group, 3-aminopropyltriethoxysilane, 3-(2-aminoethylamino)propyl trimethoxysilane, 3-(2-aminoethylamino)propyl methyl dimethoxy silane, 3-aminopropyltrimethoxysilane, and 3-phenylaminopropyltrimethoxysilane are preferable. As the dicarboxylic anhydride, phthalic anhydride, succinic anhydride, maleic anhydride, and glutaric anhydride are listed. The amount of the reaction product to be contained in the coating liquid material is favorably 1 to 10wt% and more favorably 3 to 8wt% for the first and second polysiloxanes. When the amount of the alkoxysilane having the amino group to be contained therein is less than 1wt%, the adhesion between the coating layer and the base material is insufficient. When the amount of the alkoxysilane having the amino group to be contained therein is more than 10wt%, the coating layer deteriorates in its flexibility and extensibility and thus has an insufficient adhesion to the base material, which is unpreferable.

As the third polyfunctional silane, it is preferable to use glycidoxy alkyl alkoxysilane. As the glycidoxy alkyl alkoxysilane, 3-glycidoxypropyltrimethoxysilane, 3-glycidoxypropyltriethoxysilane, 3-glycidoxypropylmethyldiethoxysilane, 3-glycidoxypropylmethyldimethoxysilane, and 2-(3,4-epoxycyclohexyl)ethyltrimethoxysilane are preferable. The amount of the third polyfunctional silane to be contained in the coating liquid material is favorably 1 to 10wt% and more favorably 3 to 8wt% for the first and second polysiloxanes. When the amount of the third polyfunctional silane to be contained therein is less than 1wt%, the adhesion between the coating layer and the base material is insufficient. When the amount of the third polyfunctional silane to be contained therein is more than 10wt%, the coating layer deteriorates in its flexibility and extensibility and thus has an insufficient adhesion to the base material, which is unpreferable.

It is preferable that the coating liquid material of the present invention does not contain "solid fine particle". The "solid fine particle" herein means a particle having a size to such an extent as to affect the roughness of the outer surface of the coating layer when the coating layer is formed by using the liquid material for medical device coating. More specifically, the "solid fine particle" means a particle having a diameter larger than the thickness of the coating layer by not less than 10%.

A catalyst for promoting heat curing may be used as an additive for the liquid material for medical device coating. As although catalysts, acid, alkali, amine, organic salts of metals, titanate, and borate are used, zinc octylate, iron octylate, and organic acid salts of cobalt, tin, lead, and the like are preferable.

As organic acid salts of tin, it is possible to use bis(2-ethyl hexanoate)tin, bis(neodecanoate)tin, di-n-butylbis(2-ethylhexyl maleate)tin, di-n-butylbis(2,4-pentanedionate)tin, di-n-(butylbutoxychloro) tin, di-n-butyl diacetoxy tin, di-n-butyltin dilaurate, dimethyltin dineodecanoate, dimethyl hydroxy(oleate)tin, and dioctyl dilauryl acid tin.

The method of producing the liquid material for medical device coating is described below.

The liquid material (emulsion) of the first polysiloxane, the liquid material (emulsion) of the second polysiloxane, and the liquid material of the polyfunctional silane are prepared.

It is possible to produce the liquid material for medical device coating by adding the liquid material (emulsion) of the first polysiloxane, the liquid material (emulsion) of the second polysiloxane, and the liquid material of the polyfunctional silane, the catalyst, and the surface active agent to purified water and mixing these components with one another. The liquid material for medical device coating produced in this manner forms an aqueous emulsion.

The medical device having slidable coating layer of the present invention is described below.

The medical device having slidable coating layer of the present invention is brought into contact with a medical member or inserted into a living body when the medical device having slidable coating layer is used. More specifically, the medical device moves in contact with an inner surface of the medical member or an inside of the living body. The medical device having slidable coating layer of the present invention has a slidable coating layer formed at a portion thereof where the medical device contacts the medical member or the inside of the living body. The slidable coating layer has a main structure containing a condensate of reactive silicone having a silanol group at both ends thereof as its main component and a large number of crosslinking parts consisting of polyfunctional silane. In addition, the slidable coating layer contains dimethylpolysiloxane having one end bonded to the crosslinking parts and the other end which does not have a reactive functional group and is not bonded to the main structure nor to the crosslinking parts. The dimethylpolysiloxane having the one end and the other end is bonded to at least one part of the crosslinking parts.

The medical device having slidable coating layer of the present invention is described below with reference to an embodiment in which the medical device having slidable coating layer is applied to a gasket for a syringe and to the syringe.

An embodiment of the gasket of the present invention is described below.

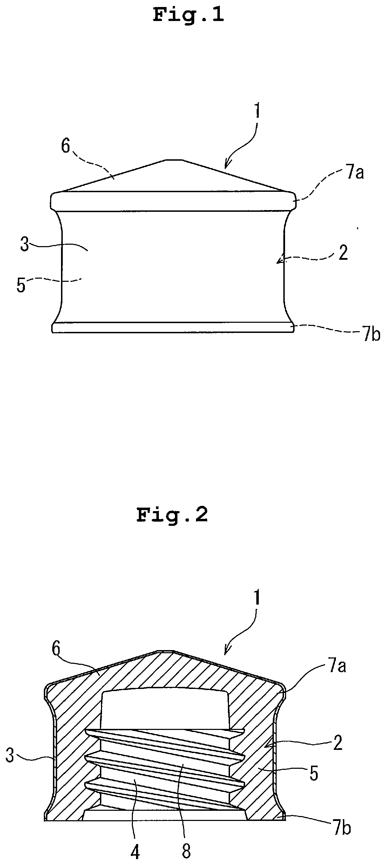

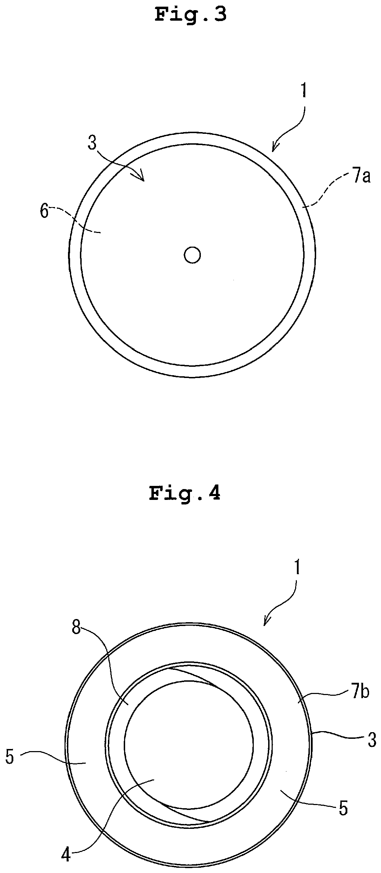

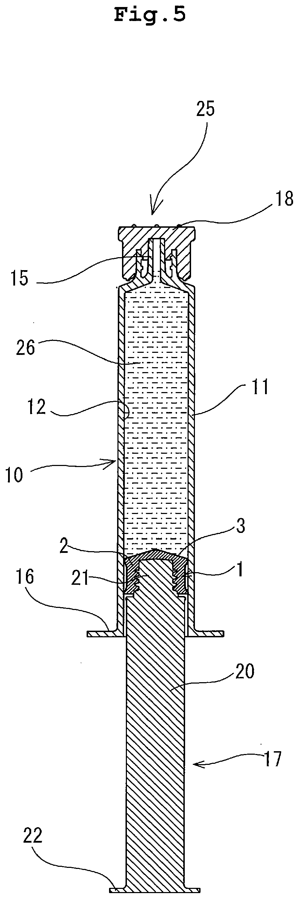

Fig. 1 is a front view showing a gasket of an embodiment of the present invention. Fig. 2 is a sectional view of the gasket shown in Fig. 1. Fig. 3 is a plan view of the gasket shown in Fig. 1. Fig. 4 is a bottom view of the gasket shown in Fig. 1. Fig. 5 is a sectional view of a prefilled syringe in which the gasket shown in Fig. 1 is used.

The medical device having slidable coating layer of this embodiment is a gasket 1, for a syringe, which is liquid-tightly and slidably accommodated inside a barrel 11, for the syringe, which is a medical member.

The gasket 1 which is the medical device having slidable coating layer of the present invention slidably contacts the inner surface of the barrel of the syringe and the slidable coating layer 3 disposed at the portion where the gasket contacts the syringe. The slidable coating layer has the main structure containing the condensate of the reactive silicone having the silanol group at both ends thereof as its main component and a large number of the crosslinking parts consisting of the polyfunctional silane. In addition, the dimethylpolysiloxane having one end bonded to the crosslinking parts and having the other end which does not have the reactive functional group and is not bonded to the main structure nor to the crosslinking parts is bonded to at least one part of the crosslinking parts.

The gasket 1 of this embodiment is used for the syringe and liquid-tightly and slidably accommodated inside the barrel 11 for the syringe. The gasket 1 has the coating layer 3 disposed at the portion thereof where the gasket 1 contacts the barrel 11. The coating layer 3 is formed as a specific coating layer to be described later. The gasket 1 has a body part (in other words, core) 2 and the slidable coating layer 3 formed at least at a portion of an outer surface of the core 2 where the gasket 1 contacts an inner surface of the barrel. The coating layer 3 may be formed on the entire outer surface of the core 2.

As shown in Figs. 1, 2, and 5, the core 2 of the gasket 1 for the syringe has a body part 5 extending in an almost equal diameter, a tapered part 6 disposed at a distal side of the body part 5 and decreasing in a tapered shape towards a distal end thereof in its diameter, a plunger-mounting part 4 disposed extendedly inside the body part 5 from a proximal end thereof toward the distal end thereof; a distal-side annular rib 7a disposed on a side surface of a distal portion of the body part 5, and a proximal-side annular rib 7b disposed on a side surface of a proximal portion of the body part 5.

As shown in Figs. 2 and 4, the plunger-mounting part 4 is formed as an approximately columnar concave portion which is disposed inside the body part 5 and extends from the proximal end of the body part 5 to a position in the vicinity of the distal end thereof. An engagedly screwing part 8 capable of engagedly screwing on a screwing part formed at a distal end of a plunger is formed on a side surface of the concave portion. A distal-end surface of the concave portion is formed almost flatly. The plunger-mounting part does not necessarily have to be formed as the engagedly screwing part, but may be formed as an engaging portion which engages the distal portion of the plunger or may be formed in combination of the engagedly screwing part and the engaging portion. An operation of mounting the plunger on the plunger-mounting part is performed by engagedly screwing the plunger on the plunger-mounting part. A state in which the engaging portion has engaged the distal portion of the plunger may be held by an engaging portion formed separately from the engagedly screwing part.

The outer diameters of the annular ribs 7a and 7b are formed a little larger than the inner diameter of the barrel 11 for the syringe. Therefore, the annular ribs 7a and 7b compressively deform inside the barrel 11. In this embodiment, two annular ribs are formed, but one or three or more annular ribs may be formed.

As materials composing the core (body part of gasket) 2, an elastic material is preferable. Although the elastic material to be used for the core 2 is not limited to a specific one, various kinds of rubber materials (specially, vulcanized rubber materials) such as natural rubber, isoprene rubber, butyl rubber, chloroprene rubber, nitrile-butadiene rubber, styrene-butadiene rubber, and silicone rubber; styrene-based elastomers and hydrogenated styrene-based elastomers; and mixtures of these styrene-based elastomers, polyolefins such as polyethylene, polypropylene, polybutene, and α-olefin copolymers, oil such as liquid paraffin, process oil, and powdery inorganic substances such as talc, cast, mica, and the like are listed.

Further it is possible to use polyvinyl chloride-based elastomers, olefin-based elastomers, polyester-based elastomers, polyamide-based elastomers, polyurethane-based elastomers, and mixtures of these elastomers as materials composing the core 2. As the composing material, the butyl rubber is preferable from the standpoint that it has elastic properties and can be sterilized by a high-pressure steam. The diene-based rubber and the styrene-based elastomers are also preferable from the standpoint that these substances can be sterilized by γ rays and electron beams.

It is essential that the slidable coating layer 3 is formed on at least the portions where the annular ribs are disposed. More specifically, it is essential that the coating layer 3 is formed at the distal-side annular rib 7a and the proximal-side annular rib 7b. The coating layer 3 may be formed on the entire outer surface of the core 2. The thickness of the coating layer 3 is favorably 1 to 30µm and especially favorably to 3 to 10µm. When the thickness of the coating layer 3 is not less than 1µm, the coating layer 3 displays a necessary slidable performance. When the thickness of the coating layer 3 is not more than 30µm, the coating layer 3 does not adversely affect the elasticity of the gasket. The coating layer 3 does not contain solid fine particles.

The slidable coating layer 3 is composed of a material having a lower friction coefficient than the above-described elastic material composing the core 2.

The slidable coating layer 3 has the main structure containing the condensate of the reactive silicone having the silanol group at both ends thereof as its main component and a large number of the crosslinking parts consisting of the polyfunctional silane. In addition, the polysiloxane having one end bonded to the crosslinking parts and having the other end which does not have the reactive functional group and is not bonded to the main structure nor to the crosslinking parts is bonded to at least one part of the crosslinking parts. It is preferable that to a certain number of the crosslinking parts, the polysiloxane having one end bonded to the crosslinking parts and having other end which does not have the reactive functional group and is not bonded to the main structure nor to the crosslinking parts is bonded. It is especially preferable that the polysiloxane having the other end which does not have the reactive functional group and is not bonded to the main structure is bonded to 5 to 50% of the crosslinking parts of the slidable coating layer.

The coating layer 3 contains a silicone compound (condensate, hardened material) as shown by a chemical formula (1) shown below and contains the silicone compound of this type as its main component.

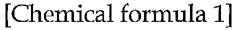

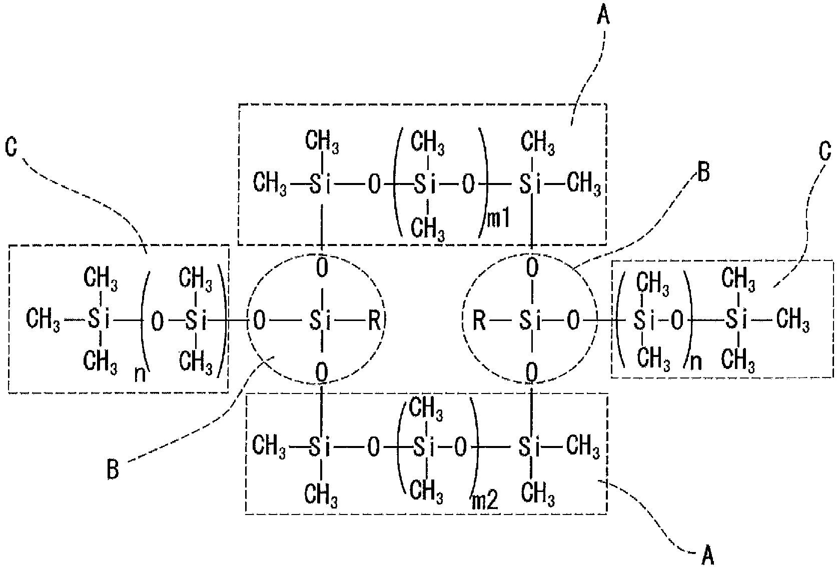

The reference symbol R shown in the chemical formula (1) denotes an alkyl group. The methyl group shown in the formula may be other alkyl groups (for example, ethyl group, propyl group or the like). It is preferable that the repeating unit n shown in the formula is integers of 40 to 680 and that the reference symbols m1 and m2 are integers of 2,700 to 6,800.

The slidable coating layer 3 is formed by applying the above-described coating liquid material to a portion of a medical device to be coated and hardening the coating liquid material. The coating layer 3 contains the silicone compound as shown by the above-described chemical formula (1) as its main component. The silicone compound shown by the chemical formula (1) has a part A which is a condensate of the second siloxane (second polysiloxane) having the silanol group at both ends thereof. The part A forms the main structure. The silicone compound (coating layer 3) shown by the chemical formula (1) has the crosslinking parts B formed of the polyfunctional silane and connecting the parts A (main structure) to each other. A large number of the crosslinking parts B are present in the coating layer 3.

Bonded to the crosslinking parts B of the silicone compound (coating layer 3) as shown by the chemical formula (1) is the polysiloxane (part C: more specifically, dimethylpolysiloxane) having one end bonded to the crosslinking parts and having the other end which does not have the reactive functional group and is not bonded to the main structure nor to the crosslinking parts B. The part C bonded to the crosslinking parts B has the silanol group at only one end thereof. The other end of the part C derives from the first siloxane (first polysiloxane) having an unreactive group.

Because the slidable coating layer 3 has the main structure and the crosslinking parts, the slidable coating layer is allowed to have a sufficiently high slidability. In addition, the slidable coating layer has a high slidability because the slidable coating layer has the above-described first polysiloxane having one end bonded to at least one part of the crosslinking parts and having the other end formed as a free end which has neither the reactive functional group (reactive group) nor is bonded to any compound.

It is preferable that the first polysiloxane is bonded to a certain number of the crosslinking parts. It is preferable that as the first polysiloxane, dimethylpolysiloxane is bonded to a certain number of the crosslinking parts. It is preferable that the other end of the dimethylpolysiloxane having the unreactive group is a trimethylsilyl group. The amount of the first polysiloxane to be bonded to the crosslinking parts is favorably 5 to 50% of the entire crosslinking parts and more favorably 10 to 40% thereof. The slidable coating layer 3 is composed of the first and second polysiloxanes bonded to the crosslinking parts. Because the amount of the crosslinking parts is ignorable, the bonding amount can be defined by a ratio occupied by the first polysiloxane.

The main structure composing the coating layer 3 may contain the dimethylpolysiloxane not having the reactive functional group (reactive group) and terminating at one end thereof. More specifically, the coating layer 3 may contain a silicone compound (condensate, hardened material) as shown by the chemical formula (2) shown below.

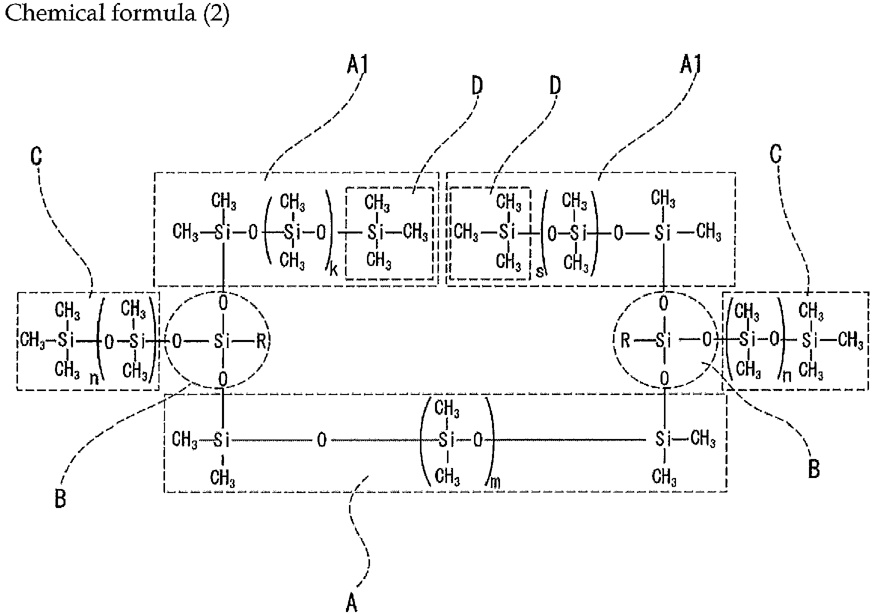

The reference symbol R shown in the chemical formula (2) denotes the alkyl group. The methyl group shown in the formula may be other alkyl groups (for example, ethyl group, propyl group or the like). It is preferable that the repeating unit n shown in the formula is integers of 40 to 680 and that the reference symbols m, k, and s are integers of 2,700 to 6,800.

The difference between the silicone compound shown by the chemical formula (2) and the silicone compound shown by the chemical formula (1) is that a part of the main structure does not consist of continuous polysiloxane having both ends thereof bonded to the polyfunctional silane, but is formed as a part A1 in which through the intermediary of the polyfunctional silane, a first siloxane (first polysiloxane) part D having the silanol group at only one end thereof and an unreactive group at the other end thereof is directly bonded (without the intermediary of the polyfunctional silane) to one end of the second siloxane (second polysiloxane) having the silanol group at both ends thereof. The other end of the first siloxane forms a free end which does not have the reactive functional group and is not bonded to any compound. The part D allows the coating layer to have a high slidability. In the coating layer 3 containing the chemical formula 2, the main structure contains the polysiloxane (for example, dimethylpolysiloxane) which does not have the reactive functional group and terminates at one end thereof.

The ratio (weight ratio) between the part A and the part A1 in the chemical formula (2) is favorably 50:50 to 95:5 and more favorably 60:40 to 90:10. Because the part A is present in the silicone compound to some extent, the coating layer has a sufficiently high strength. Because the part A1 is present in the silicone compound to some extent, the part A1 imparts a high slidability to the coating layer.

As shown in a chemical formula (3), the slidable coating layer 3 has the main structure containing the condensate of the reactive silicone having the silanol group at both ends thereof as its main component and a large number of the crosslinking parts formed of the polyfunctional silane. In addition, the slidable coating layer may contain a structure in which a plurality of dimethylpolysiloxanes which do not have the reactive functional group and terminate at one end thereof are bonded to one part of the polyfunctional silane.

The reference symbol R shown in the chemical formula (3) denotes the alkyl group. The methyl group shown in the chemical formula may be other alkyl groups (for example, ethyl group, propyl group or the like). The repeating unit shown in the chemical formula is as shown in the chemical formulas (1) and (2).

It is preferable that the coating layer 3 formed on the gasket of the present invention does not contain "solid fine particle". The "solid fine particle" herein means a particle having a size to such an extent as to affect the roughness of the outer surface of the coating layer 3 when the coating layer 3 is formed. More specifically, the "solid fine particle" means a particle having a diameter larger than the thickness of the coating layer by not less than 10%.

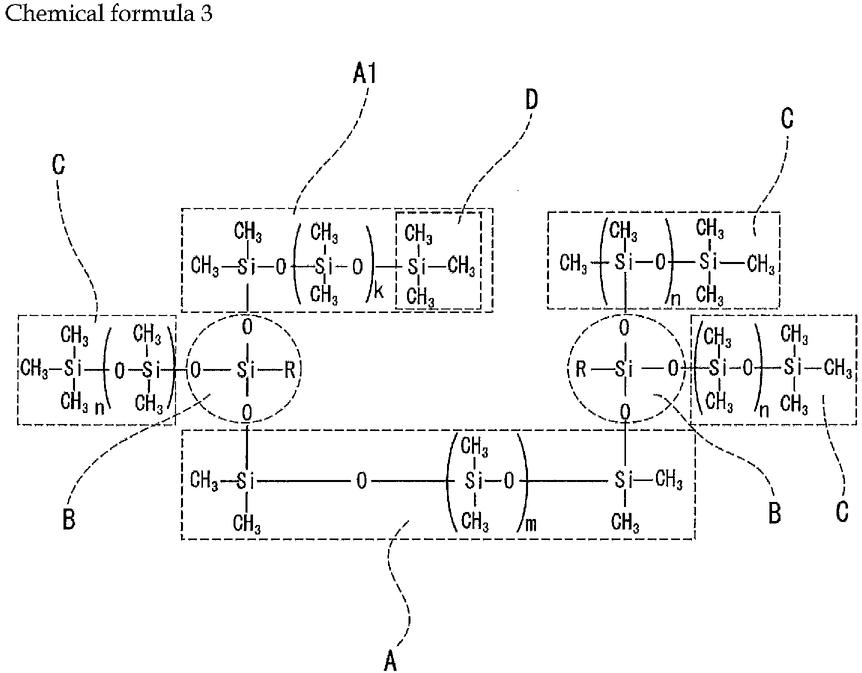

The method of forming the coating layer 3 is described below.

In the method of forming the coating layer, a coating layer is obtained by applying a coating solution to a clean surface of the gasket and thereafter hardening it. At this time, as the method of applying the coating solution to the surface of the gasket, it is possible to use known methods such as a dipping method, a spraying method, and the like. It is especially preferable to apply the coating solution as a spray (spray application) to the surface of an object to be coated with the object being rotated (specifically, at 100 to 600 rpm). In applying the coating solution as a spray to the surface of the gasket, it is preferable to do so after heating a portion of the gasket to be coated to 60 to 120 degrees C. Thereby, the coating solution rapidly fixes to the surface of the gasket to be coated without water repellency.

As the method of hardening the coating solution, it may be left at a normal temperature, but it is preferable to harden the coating solution by heating it. The method of thermally hardening the coating solution is not limited to a specific method, provided that the base material of the gasket is not modified or deformed. Hot-air drying, and a drying oven using infrared rays, and the like are exemplified. It is also possible to use a known method such as a method of using a decompression drier. The thickness of the coating layer to be formed is 1 to 30µm and more favorably 3 to 10µm. Such a coating layer can be easily formed by appropriately controlling the concentration of a mixed solution, the dipping method, and the spraying method.

The syringe 10 of the present invention has a barrel 11, a gasket 1 slidably accommodated inside the barrel 11, and a plunger 17 which has been mounted on the gasket 1 or can be mounted thereon.

More specifically, as shown in Fig. 5, the syringe 10 is constructed of the barrel 11, for use in the syringe, which has a needle-mounting portion 15 disposed at the distal part thereof and a pair of opposed flanges 16 disposed at the proximal end thereof; the gasket 1, for use in the syringe, which is capable of liquid-tightly and airtightly sliding on an inner surface 12 of the barrel 11 for use in the syringe; the plunger 17 which has been mounted on the gasket 1 for use in the syringe or can be mounted thereon; a sealing member 18 for sealing the needle-mounting portion 15 of the barrel 11 for use in the syringe; and a medical agent accommodation portion, for accommodating a medical agent 26, which is formed among the sealing member 18, the inner surface 12 of the barrel 11, and the gasket 1 for use in the syringe.

Instead of the sealing member 18, an injection needle may be mounted on the needle-mounting portion 15. In the syringe of this embodiment, as the sealing member, an elastic cap, as shown in Fig. 5, removable from the needle-mounting portion is used. As sealing member, the sealing member may be directly insertable into thereof by a double-ended needle.

The gasket 1 has the above-described coating layer 3 formed on its surface. In the syringe 10, it is preferable that the dynamic sliding resistance value of the gasket 1 when the gasket 1 slides inside the barrel 11 at a low speed (100mm/minute) is not more than 20N. Such a low dynamic sliding resistance value can be obtained by forming the above-described coating layer 3 on the surface of the gasket 1. It is especially preferable that the dynamic sliding resistance value of the gasket 1 when the gasket 1 slides inside the barrel 11 at the low speed (100mm/minute) is 1N to 20N.

This medical device is a prefilled syringe 25 composed of the syringe 10 and the medical agent 26, as shown in Fig. 5.

The barrel 11 for use in the syringe is a cylindrical member having the needle-mounting portion 15 disposed at the distal part thereof and the flange 16 disposed at the proximal end thereof. The barrel 11 is made of a material transparent or semitransparent. It is preferable that the barrel 11 is made of a material having a low oxygen permeability or a low vapor permeability. It is preferable that the material forming the barrel 11 has a glass transition point or a melting point not less than 110 degrees C.

As materials forming the barrel 11, various general-purpose rigid plastic materials, for example, polyolefins such as polypropylene, polyethylene, poly (4-methylpentene-1), and cyclic polyolefin; polyesters such as polyethylene terephthalate, polyethylene naphthalate, and non-crystalline polyarylate; polystyrene; polyamide; polycarbonate, polyvinyl chloride; acrylic resin; an acrylonitrile-butadiene-styrene copolymer, and non-crystalline polyetherimide are preferable. The polypropylene, the poly (4-methylpentene-1), the cyclic polyolefin, the polyethylene naphthalate, and the non-crystalline polyetherimide are especially preferable because these resins are transparent and resistant to heat sterilization. In addition to the barrel, these resins can be commonly used as materials to form containers capable of accommodating medical agents. It is also possible to use glass as a material to form the barrel.

As shown in Fig. 5, the plunger 17 has a sectionally cross-shaped body part 20 extended axially; a plunger-side screwing part 21, disposed at the distal part thereof, which screws on the plunger-mounting part 4; a disk-shaped gasket-supporting part disposed between the plunger-side screwing part 21 and the body part 20; a disk part 22, for pressing use, which is disposed at the proximal end of the body part 20; and a disk-shaped rib (not shown) formed midway on the body part 20.

The medical agent 26 is accommodated inside the syringe 10 of this embodiment. As the medical agent 26, it is possible to use both a liquid medicine and a solid agent such as a powdery medical agent and a freeze-dried medical agent. The liquid medicine, containing the surface active agent, which has a low viscosity and a high degree of penetration is preferable because although the liquid medicine makes it difficult to allow the gasket to have slidability and to be liquid-tight, the liquid medicine can be preferably accommodated inside the syringe 10 which does not require silicone oil. When a biotechnology-based medical agent is accommodated inside the syringe as a medical agent, silicone oil-caused denaturation thereof does not occur because the silicone oil is not used. Even though the medical agent has a high viscosity, the medical agent can be favorably discharged from the syringe because the gasket has a low sliding resistance value and a stable slidability.

In the case where the coating layer 3 is formed on the gasket 1 for the syringe at the part thereof where the gasket contacts the accommodated medical agent, it is possible to prevent the adsorption of the medical agent such as the liquid medicine which contains a component having a poor water solubility and has a high adsorbing property. Thus, it is preferable to use such a medical agent.

As materials composing the plunger 17 and the sealing member 18, it is preferable to use hard resins or semi-hard resins such as polyvinyl chloride, high-density polyethylene, polypropylene, polystyrene, polyethylene terephthalate, polycarbonate, acrylic resin, and the like.

The above-described syringe is an example of the medical device which moves in contact with the inner surface of the medical member. This type of the medical device is not limited to the syringe, but may be any medical devices, provided that they slidably contact the inside of the medical member. For example, this type of the medical device may be a rubber stopper-provided vial container, a transfusion bag, a blood collection tube, and a decompression blood collection tube. The medical device of the present invention is not limited to the gasket for the syringe, but may be any of an O-ring, a stopper, a cover, and the like, provided that they slidably contact the medical member. For example, the medical device of the present invention may be a rubber stopper of the vial container, a lid of the transfusion bag, and the like.

The medical device of the present invention may be an appliance to be inserted into a living body. As such a medical device to be inserted into the living body, a catheter, a guide wire, a blood vessel dilation appliance, and the like are known. The medical device of the present invention moves in contact with the inside (for example, blood vessel, the inner surface of the digestive tube, the outer surfaces of internal organs) of the living body when the medical device is inserted into the living body. As such a medical device which moves in contact with the inside of the living body, catheter, a guide wire, a blood vessel dilation appliance, and the like which are inserted into the catheter (for example, a guiding catheter) which is a medical member to guide the distal portions thereof to an intended portion inside the living body.

An embodiment in which the medical device of the present invention is applied to the guide wire is described below with reference to drawings.

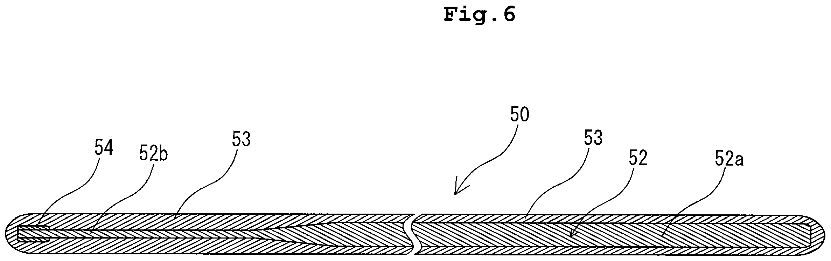

Fig. 6 is a sectional view of one embodiment of the guide wire of the present invention.

A guide wire 50 of this embodiment has an inner core 52 and a slida ble coating layer 53 enclosing the inner core 52. The slidable coating layer 53 is composed of silicone rubber and contains carbon nanotubes and silicon e-based resin fine particles.

The guide wire of the embodiment shown in Fig. 6 has the inner core 52 composed of the body part 52a having a high rigidity and the distal part 52b, having a smaller diameter and a lower rigidity than the body part 52a, which is formed integrally with the body part 52a, a high radiographic visualization part 54 formed at the distal end of the inner core 52, and the slidable coating layer 53 enclosing the entire inner core 52 on which the high radiographic visualization part 54 is formed. Because the coating layer contains fine particles, it has a rough surface.

The inner core 52 of the guide wire 50 has the body part 52a and the distal part 52b and is integrally formed of an elastic metal. The diameter of the distal part 52b is so formed as to be smaller than the distal end of the body part 52a. By so forming the distal part 52b as to have a small diameter, the distal part 52b has a lower rigidity than the body part. The diameter of the distal part 52b may be so set as to become gradually smaller toward the distal end thereof from the distal end of the body part 52a. By making the distal part of the inner core gradually smaller in its diameter, the distal part of the inner core gradually bends when a force is applied to the distal end of the body part 52a. Thus, operability is improved.

It is preferable that the inner core 52 is made of a superelastic metal and stainless steel. As the superelastic metal, superelastic metallic bodies such as a TiNi alloy containing 49-58 at% Ni, a Cu-Zn alloy containing 38.5 to 41.5wt% Zn, a Cu-Zn-X alloy containing 1 to 10wt% X (X=Be, Si, Sn, Al, Ga), and a Ni-Al alloy containing 36 to 38 at% Al are preferably used. The TiNi alloy is especially preferable.

The outer diameter of the body part 52a of the inner core 52 is favorably 0.10 to 1.00mm and more favorably 0.15 to 0.40mm. The length of the body part is favorably 1000 to 4000mm and more favorably 1500 to 3000mm. The buckling strength (yield stress when a load is applied) of the body part is favorably 30 to 100 Kg/mm²(22 degrees C) and more favorably 40 to 55Kg/mm². The restoration stress (yield stress when a load is removed) of the body part is favorably 20 to 80 Kg/mm²(22 degrees C) and more favorably 30 to 35 Kg/mm².

The outer diameter of the distal part 52b of the inner core 52 is favorably 0.03 to 0.15mm and more favorably 0.05 to 0.10mm. The length of the distal part of the inner core is favorably 10 to 300mm and more favorably 50 to 150mm. The bending load of the distal part is favorably 0.1 to 10g and more favorably 0.3 to 6.0g. The restoration load of the distal part is favorably 0.1 to 10g and more favorably 0.3 to 6.0g.

The outer diameter of the distal part of the inner core do not necessarily have to be set to the above-described range, but may be so set as to satisfy a part of the above-described range. Further the restoration stress of the body part and that of the distal part do not necessarily have to have an equal value, but it is preferable to make a device to allow the restoration stress of the body part and that of the distal part to be differentiated from each other by heat-treating them in different conditions so that the body part and the distal part have an appropriate wire diameter and thus an appropriate property respectively. That is, it is preferable to heat-treat the body part and the distal part in different conditions to allow the restoration stress of the body part to be high and that of the distal part to be flexible. In addition, the inner core 52 does not necessarily have to be composed of a single wire, but may be composed of a plurality of parallel or twisted wires so that the inner core displays the above-described function, namely, a stepwise change or a continuous change of physical properties.

In the example shown in Fig. 6, the high radiographic visualization part 54 is a metallic annular member, having a high radiographic visualization performance, which is fixed to the distal end of the inner core 52. Specifically, the high radiographic visualization part is formed of a pipe-shaped member. As metals having high radiographic visualization performance, gold, platinum, zinc, silver, bismuth, and tungsten are favorable. Gold is especially favorable.

The high radiographic visualization part 54 is fixed to the distal end of the inner core 52 by mechanically crimping the high radiographic visualization part to the distal end thereof or by soldering the high radiographic visualization part to a plated or evaporated metal.

The outer diameter of the high radiographic visualization part 54 is 0.20 to 0.90mm and preferably 0.25 to 0.40mm. The inner diameter thereof is 0.04 to 0.16mm and preferably 0.06 to 0.11mm. The length thereof is 1.00 to 10.00mm and preferably 1.5 to 4.0mm.

The high radiographic visualization part 54 may be composed of a coiled thin wire formed of the above-described metal having high radiographic visualization performance. The thin wire having a diameter of 0.02 to 0.10mm can be preferably used. The length of the high radiographic visualization part to be wound on the distal end of the inner core is 1.0 to 10.0mm and preferably 1.5 to 4.0mm from the distal end thereof.

As shown in Fig. 6, it is preferable that the slidable coating layer 53, including the distal part thereof, which coats the entire inner core 52 has an almost uniform outer diameter. The slidable coating layer 53 has an almost uniform outer diameter to prevent the difference in level formed at the distal end of the inner core 52 between the inner core 52 and the high radiographic visualization part 54 from affecting the outer configuration of the guide wire 50.

The same material as that of the coating layer 3 described on the gasket of the above-described embodiment can be preferably used as the material of the slidable coating layer 53.

The outer diameter of the slidable coating layer is 0.25 to 1.04mm and preferably 0.30 to 0.64mm. The thickness of the slidable coating layer at the body part 52a of the inner core 52 is 0.25 to 1.04mm and preferably 0.30 to 0.64mm.

It is preferable that the distal end (the distal end of the slidable coating layer 53) of the guide wire 50 has a curved surface, for example, a semispherical surface as shown in Fig. 6 to prevent a blood vessel wall from being damaged and improve the operability of the guide wire 50.

Although the entire inner core 52 of the guide wire 50 of this embodiment is coated with the slidable coating layer 53, the form of the inner core 52 is not limited to this one. The slidable coating layer 53 may be so constructed as to cover only a part of the inner core 52. For example, the slidable coating layer 53 may be so formed as to cover only the distal part of the inner core 52 or only the body part of the inner core 52.

### EXAMPLES

Examples of the present invention are described below.

### Example 1

### 1a) Polydimethylsiloxane having silanol group at its one end and trimethylsilyl at its other end as its main components was prepared as described below.

200g of octamethylcyclotetrasiloxane, 2g of hexamethyldisiloxane, 160g of a 10% dodecylbenzene sulfonate aqueous solution were mixed with one another in a tall beaker. After the mixture was stirred by a homomixer, emulsifying treatment was performed by using a high-pressure emulsifying device. After an emulsified liquid was heated at 70 degrees C for six hours, the emulsified liquid was incubated at 15 degrees C for 18 hours to obtain an emulsion. After the emulsion was destroyed, an extract was subjected to GPC analysis to obtain the polydimethylsiloxane having a molecular weight of approximately 30,000.
2) Polydimethylsiloxane whose main components are both-end silanol was prepared as described below.

200g of octamethylcyclotetrasiloxane, 160g of a 10% dodecylbenzene sulfonate aqueous solution were mixed with each other in a tall beaker. After the mixture was stirred by a homomixer, emulsifying treatment was performed by using a high-pressure emulsifying device. After an emulsified liquid was heated at 70 degrees C for six hours, the emulsified liquid was incubated at 15 degrees C for 18 hours to obtain an emulsion. After the emulsion was destroyed, an extract was subjected to the GPC analysis to obtain the polydimethylsiloxane having a molecular weight of approximately 300,000.

The following products were prepared.
3) Product name: Z-6366 (produced by Dow Corning Toray Co., Ltd.) containing methyltrimethoxysilane as its main component
4) Mixture of product name Z-6011 (produced by Dow Corning Toray Co., Ltd.) containing 3-aminopropyltriethoxysilane as its main component and an ethanol solution of maleic anhydride (resin ratio: 50%)
5) Product name Z-6040 (produced by Dow Corning Toray Co., Ltd.) containing 3-glycidoxypropyltrimethoxysilane as its main component
6) Dioctyltin dilaurate (catalyst)
7) Straight-chain sodium alkylbenzene sulfonate (surface active agent)

20 parts by weight of the above-described 1a) (weight of emulsion/solid content: 50% polydimethylsiloxane having the silanol group at its one end and the trimethylsilyl at its other end), 37.6 parts by weight of the above-described 2) (weight of emulsion/solid content: 50% polydimethylsiloxane having the silanol group at its both ends), 0.2 parts by weight of the above-described 3), 1.7 parts by weight of the above-described 4), 0.9 parts by weight of the above-described 5), 0.3 parts by weight of the above-described 6), and 0.4 parts by weight of the above-described 7) were added to 39 parts by weight of purified water. Thereafter the mixture was stirred to prepare a coating liquid material (example 1) of the present invention.

### Example 2

### 1b) Polydimethylsiloxane containing silanol at its one end and trimethylsilyl at its other end as its main components was prepared as described below.

200g of octamethylcyclotetrasiloxane, 10g of hexamethyldisiloxane, 160g of a 10% dodecylbenzene sulfonate aqueous solution were mixed with one another in a tall beaker. After the mixture was stirred by a homomixer, emulsifying treatment was performed by using a high-pressure emulsifying device. After an emulsified liquid was heated at 70 degrees C for six hours, the emulsified liquid was incubated at 15 degrees C for 18 hours to obtain an emulsion. After the emulsion was destroyed, an extract was subjected to GPC analysis to obtain the polydimethylsiloxane having a molecular weight of approximately 8,000.

20 parts by weight of the above-described 1b) (weight of emulsion/solid content: 50% polydimethylsiloxane having the silanol group at its one end and the trimethylsilyl at its other end), 37.6 parts by weight of the above-described 2) (weight of emulsion/solid content: 50% polydimethylsiloxane having the silanol group at its both end), 0.2 parts by weight of the above-described 3), 1.7 parts by weight of the above-described 4), 0.9 parts by weight of the above-described 5), 0.3 parts by weight of the above-described 6), and 0.4 parts by weight of the above-described 7) were added to 39 parts by weight of purified water. Thereafter the mixture was stirred to prepare a coating liquid material (example 2) of the present invention.

### Example 3

### 1c) Polydimethylsiloxane containing the silanol group at its one end and the trimethylsilyl group at its other end as its main components was prepared as described below.

200g of octamethylcyclotetrasiloxane, 20g of hexamethyldisiloxane, 160g of a 10% dodecylbenzene sulfonate aqueous solution were mixed with one another in a tall beaker. After the mixture was stirred by a homomixer, emulsifying treatment was performed by using a high-pressure emulsifying device. After an emulsified liquid was heated at 70 degrees C for six hours, the emulsified liquid was incubated at 15 degrees C for 18 hours to obtain an emulsion. After the emulsion was destroyed, an extract was subjected to GPC analysis to obtain the polydimethylsiloxane having a molecular weight of approximately 4,000.

20 parts by weight of the above 1c) (weight of emulsion/solid content: 50% polydimethylsiloxane having the silanol group at its one end and the trimethylsilyl at its other end), 37.6 parts by weight of the above-described 2) (weight of emulsion/solid content: 50% polydimethylsiloxane having the silanol group at its both ends), 0.2 parts by weight of the above-described 3), 1.7 parts by weight of the above-described 4), 0.9 parts by weight of the above-described 5), 0.3 parts by weight of the above-described 6), and 0.4 parts by weight of the above-described 7) were added to 39 parts by weight of purified water. Thereafter the mixture was stirred to prepare a coating liquid material (example 3) of the present invention.

### Example 4

3.3 parts by weight of the above 1a) (weight of emulsion/solid content: 50% polydimethylsiloxane having the silanol group at its one end and the trimethylsilyl at its other end), 37.6 parts by weight of the above-described 2) (weight of emulsion/solid content: 50% polydimethylsiloxane having the silanol group at its both ends), 0.2 parts by weight of the above-described 3), 1.7 parts by weight of the above-described 4), 0.9 parts by weight of the above-described 5), 0.3 parts by weight of the above-described 6), and 0.4 parts by weight of the above-described 7) were added to 39 parts by weight of purified water. Thereafter the mixture was stirred to prepare a coating liquid material (example 4) of the present invention.

### Example 5

33 parts by weight (weight of emulsion/solid content: 50% polydimethylsiloxane having the silanol group at its one end and the trimethylsilyl at its other end) of the above 1a), 37.6 parts by weight (weight of emulsion/ solid content: 50% polydimethylsiloxane having the silanol group at its both ends) of the above-described 2), 0.2 parts by weight of the above-described 3), 1.7 parts by weight of the above-described 4), 0.9 parts by weight of the above-described 5), 0.3 parts by weight of the above-described 6), and 0.4 parts by weight of the above-described 7) were added to 39 parts by weight of purified water. Thereafter the mixture was stirred to prepare a coating liquid material (example 5) of the present invention.

### Comparison example 1

Without using the polydimethylsiloxane, prepared in the examples 1 through 3, which contains the silanol group at its one end and the trimethylsilyl group at its other end, 56.4 parts by weight (weight of emulsion/solid content: 50% polydimethylsiloxane having the silanol group at its both ends) of the above 2) of the example 1, 0.3 parts by weight of the above-described 3), 2.6 parts by weight of the above-described 4), 1.3 parts by weight of the above-described 5), 0.4 parts by weight of the above-described 6), and 0.7 parts by weight of the above-described 7) were added to 38.5 parts by weight of purified water. Thereafter the mixture was stirred to prepare a coating liquid material (comparison example 1).

### Comparison example 2

Without using the polydimethylsiloxane, prepared in the examples 1 through 3, which contains the silanol group at its one end and the trimethylsilyl group at its other end, 20 parts by weight of silicone oil (product name: DOW CORNING (R) 360 MEDICAL FLUID 1000cSt (produced by Dow Corning Toray Co., Ltd.), 37.6 parts by weight (weight of emulsion/solid content: 50% polydimethylsiloxane having the silanol group at its both ends) of the above 2) of the example 1, 0.2 parts by weight of the above-described 3), 1.7 parts by weight of the above-described 4), 0.9 parts by weight of the above-described 5), 0.3 parts by weight of the above-described 6), and 0.4 parts by weight of the above-described 7) were added to 39 parts by weight of purified water. Thereafter the mixture was stirred to prepare a coating liquid material (comparison example 2).

### Example 6

The core, of the gasket for the syringe, having the configuration shown in Figs. 1 and 2 was made by using butyl rubber. The core was formed by press-molding a vulcanizable rubber composition composed of butyl rubber to which an additive was added. Regarding the configuration of the obtained core, the length thereof was 20mm; the outer diameter thereof at the distal-side and proximal-side annular ribs thereof was 23.7mm; the length between the center of the distal-side annular rib and that of the proximal-side annular rib was 10mm; the outer diameter of the core at an equal-diameter portion thereof between the distal-side and proximal-side annular ribs was 21.5mm; the length (depth) of the plunger-mounting part having a female screw at the inner side thereof was 8mm; and the inner diameter of a plunger-mounting concave portion at its distal side and proximal side were 14.5mm and 15mm respectively.

After the core material of the gasket made as described above in an environment having a room temperature and a normal pressure were heat-treated at 90 degrees C for 30 minutes, the gasket was rotated (300 rpm) on the axis thereof, and the coating liquid material of the example 1 was sprayed to the gasket from the side surface thereof. Thereafter the coating liquid material was dried at 150 degrees C for 30 minutes. In this manner, the gasket having the coating layer was made. Thereafter to wash an extra coating liquid present on the gasket, cleaning was performed with purified water having a temperature not less than 80 degrees C. The average thickness of the coating layer formed on the surface of the core material was about 8µm. In this way, the gasket of the example 6 was obtained.

### Example 7

Except that the coating liquid material of the example 2 was used as the coating liquid material of the example 7, a gasket (example 7) of the present invention was prepared in a manner similar to that of the example 6.

### Example 8

Except that the coating liquid material of the example 3 was used as the coating liquid material of the example 8, a gasket (example 8) of the present invention was prepared in a manner similar to that of the example 6.

### Example 9

Except that the coating liquid material of the example 4 was used as the coating liquid material of the example 9, a gasket (example 9) of the present invention was prepared in a manner similar to that of the example 6.

### Example 10

Except that the coating liquid material of the example 5 was used as the coating liquid material of the example 10, a gasket (example 10) of the present invention was prepared in a manner similar to that of the example 6.

### Comparison example 3

Except that the coating liquid material of the comparison example 1 was used as the coating liquid material of the comparison example 3, a gasket (comparison example 3) was prepared in a manner similar to that of the example 6.

### Comparison example 4

Except that the coating liquid material of the comparison example 2 was used as the coating liquid material of the comparison example 4, a gasket (comparison example 4) was prepared in a manner similar to that of the example 6.

### Experiment 1: Sliding Resistance Measurement Test

Polypropylene (produced by Japan Polychem Corporation) used as a material for forming barrels for syringes was injection-molded to form the barrels for the syringes each having a configuration shown in Fig. 5. The cylindrical portion of each of the barrels for the syringes had an inner diameter of 23.5mm and a length of 95mm. The polypropylene (produced by Japan Polychem Corporation) used as a material for forming plungers was injection-molded to form the plungers each having the configuration shown in Fig. 5.

The above-described barrels for the syringes, the gaskets of the examples 6 through 10 and the comparison examples 3 and 4, and the above-described plungers were assembled to form the syringes.

The sliding resistance value of each syringe was measured by using an autograph (model name: EZ-Test, manufactured by Shimazu Seisakusho Co., Ltd.). More specifically, with the distal end of each syringe and the proximal end of the plunger being fixed to a fixing portion of the autograph to which an object to be measured is fixed, the plungers were moved downward 60mm at a speed of 100mm/minute to measure the initial sliding resistance value and maximum sliding resistance value (N) of each syringe. The results are as shown in table 1.

### Experiment 2: Peel-off Test

A test described below was conducted on syringes prepared by using the gaskets of the examples 6 through 10 and the comparison examples 3 and 4 prepared in a manner similar to that of the experiment 1.

Each syringe was evacuated from the cylinder tip thereof. When the degree of vacuum inside the syringe reached not less than -90 kPa, a stopper was slid along the inner wall of the syringe. Whether a substance which peeled off the coating layer was present on the inner wall of the syringe and on the surface of the stopper was checked. The results are as shown in table 1.

The number of samples used in the test was five. A mark of "○" was given to the examples and the comparison example where the substance which peeled off the coating layer was detected in none of the samples.

### Experiment 3: Pressure Test Specified in Standard of Sterilized Syringe Cylinder

A test described below was conducted on syringes prepared by using the gaskets of the examples 6 through 10 and the comparison examples 3 and 4 prepared in a manner similar to that of the experiment 1.

The test conducted conformed to the pressure test specified in the standard of a sterilized plastic syringe cylinder which can be immediately used as it is and should be disposed after using it one time (notified on December 11, 1998 by Director of Pharmaceutical and Medical Safety Bureau in No. 1079 issue of Pharmaceutical Development in Japan). The results are as shown in table 1.

The number of samples used in the test was five. The mark of "○" was given to the examples and the comparison example in which all of the five samples conformed to the standard.

### Experiment 4: Sealing Test

The same barrels for syringes as those used in the experiment 1 were prepared. After TSB culture medium was filled into the barrels for syringes, the gaskets of the examples 6 through 10 and the comparison examples 3 and 4 were plugged to prepare TSB-filled syringes. Thereafter the syringes were subjected to autoclave sterilization.

Test bacteria Serratia marcescens were diluted in the TSB culture media in such a way that the amount thereof became not less than 10⁶cfu/mL to prepare a test bacterium solution.

Thereafter the test bacterium solution was injected into a beaker until an end surface of each gasket of each sterilized syringe in which the TSB culture medium was filled was immersed in the test bacterium liquid. After the syringe was allowed to stand inside a pressure jar, a pressure higher than the atmospheric pressure by 0.02 MPa was applied to the pressure jar for 30 minutes by a pressure pump. After the pressure application finished, each sterilized syringe in which the TSB culture medium was filled was cleaned with purified water and disinfected with 70% ethanol. After the test bacteria were cultured at 31±1 degree C for 14 days, whether bacteria grew in the TSB culture media was checked according to weather the TSB culture media were cloudy. TSB culture media not cloudy were marked by "○". The results are as shown in table 1.

The number of samples used in the test was five. The mark of "○" was given to the examples and the comparison example in which all of the five samples were acceptable.

### Experiment 5: Fixing Test

Plates were prepared by using polypropylene (produced by Japan Polychem Corporation) having a dimension of 50mm×70mm, thickness: 2mm as a material for forming the plates. After rubber sheets (10mm×50mm, thickness: 15mm) made of butyl rubber same as the material for the cores of the gaskets of the examples and the comparison examples were heat-treated at 90 degrees C for 30 minutes, the coating liquid materials of the examples 1 through 5 and the comparison examples 1 and 2 were sprayed to the rubber sheets respectively. Thereafter the coating liquid materials were dried at 150 degrees C for 30 minutes to prepare specimens.

Each specimen was sandwiched between the polypropylene plate and an iron plate with a coated surface of the specimen facing the polypropylene plate and fixed to the polypropylene plate and the iron plate with clips. Thereafter the specimens were allowed to stand for one day in a constant-temperature bath whose temperature was set to 40 degrees C, 60 degrees C, and 80 degrees C and thereafter for 10 days, 20 days, and 30 days in the constant-temperature bath whose temperature was set to 60 degrees C. After the specimens were allowed to stand in the above-described manner, fixing degrees of the specimens were measured by using an autograph (model name: EZ-Test, produced by Shimazu Seisakusho Co., Ltd.). The results are as shown in table 1.

**Table 1**

| | Sliding resistance value(N) | | Peel-off test | Pressure test | Sealing test | Fixing test |
|---|---|---|---|---|---|---|
| | Initial stage | Maximum | | | | |
| Example 6 | 2.4 | 5.6 | ○ | ○ | ○ | ○ |
| Example 7 | 4.1 | 5.8 | ○ | ○ | ○ | ○ |
| Example 8 | 5.5 | 6.0 | ○ | ○ | ○ | ○ |
| Example 9 | 5.5 | 6.5 | ○ | ○ | ○ | ○ |
| Example 10 | 3.2 | 5.5 | ○ | ○ | ○ | ○ |
| Comparison example 3 | 6.5 | 8.5 | ○ | ○ | ○ | ○ |
| Comparison example 4 | 2.0 | 2.0 | ○ | ○ | ○ | ○ |

### Experiment 7: Silicone Extraction Test

10 gaskets of each of the examples 6 through 10 and the comparison examples 3 and 4 were put into cylindrical filter paper respectively and refluxed in a hexane solvent for eight hours. After an extracted liquid was concentrated by an evaporator, it was dried under a decreased pressure. Thereafter collected materials were subjected to GPC measurement to check whether a peak corresponding to the molecular weight of each silicone extract was present. Results are as shown in table 2. The number of samples used in the test was five. A mark of "○" was given to the examples and the comparison example in which the peak corresponding to the molecular weight of the silicone extract was detected in none of the samples, whereas "×" was given to the examples and the comparison example in which the peak corresponding to the molecular weight of the silicone extract was detected in the samples.

### Experiment 8: Medical Agent Coagulation Test

After erythropoietin (produced by Sigma-Aldrich Corporation) was added to an aqueous solution containing 2mM of Na₂HPO₄ and 0.06mg/mL of polysorbate 80, the erythropoietin was completely dissolved therein to prepare a solution (erythropoietin solution formulation) in which the concentration of the erythropoietin was 24,000 IU/mL.

The same gasket for the barrel for the syringe as that used in the experiment 1 was prepared. After an opening at the distal end of the barrel was sealed with a sealing cap made of butyl rubber, 2mL of the erythropoietin solution formulation was filled in the syringe. Thereafter gaskets of the examples 6 through 10 and the comparison examples 3 and 4 were plugged to prepare prefilled syringes.

After prefilled syringes, prepared as described above, which were filled with the erythropoietin solution formulation were stored at four degrees C for 4, 8, and 12 weeks, each erythropoietin solution formulation taken out of the syringes was evaluated in its coagulation state. The coagulation state was evaluated by using the results obtained by measuring nanoparticles by NTA (Nanoparticle Tracking Analysis), micro particles by Flow CAM, and particle size distribution by DLS (Dynamic Light Scattering), and the molecular weight of the erythropoietin by SEC-MALS (Size Exclusion Chromatography-Multi Angle Light Scattering).

The results are as shown in table 2. The number of samples used in the test was five. A mark of "○" was given to the examples and the comparison example in which the coagulation of the erythropoietin was detected in none of the samples.

**Table 2**

| | Silicone extraction test | Medical agent coagulation test | | |
|---|---|---|---|---|
| | | 4 weeks | 8 weeks | 12 weeks |
| Example 6 | ○ | ○ | ○ | ○ |
| Example 7 | ○ | ○ | ○ | ○ |
| Example 8 | ○ | ○ | ○ | ○ |
| Example 9 | ○ | ○ | ○ | ○ |
| Example 10 | ○ | ○ | ○ | ○ |
| Comparison example 3 | ○ | ○ | ○ | ○ |
| Comparison example 4 | × | × | × | × |

The liquid material for medical device coating of the present invention has the form described below.
(1) A liquid material for medical device coating for imparting slidability to said medical device, wherein said liquid material for medical device coating contains first polysiloxane having a silanol group only at one end thereof, second polysiloxane having said silanol group at both ends thereof, and polyfunctional silane.

The slidable coating layer is formed by applying the liquid material for medical device coating to a portion of the medical device having slidable coating layer where the medical device contacts the medical member or a lumen and heat-curing the liquid material for medical device coating. The slidable coating layer formed in the above-described manner has the main structure containing the condensate of the reactive polysiloxane, in other words, the second polysiloxane having the silanol group at both ends thereof as the main component thereof and a large number of the crosslinking parts formed of the polyfunctional silane. In addition, the slidable coating layer has the above-described first polysiloxane having one end bonded to at least one part of the crosslinking parts and having the other end formed as the free end which has neither the reactive functional group nor is bonded to any compound. Because the slidable coating layer has the above-described main structure and the crosslinking parts, the slidable coating layer is allowed to have a sufficiently high slidability. In addition, the slidable coating layer has the high slidability because the slidable coating layer has the above-described first polysiloxane having one end bonded to at least one part of the crosslinking parts and having the other end formed as the free end which has neither the reactive functional group nor is bonded to any compound.

The above-described embodiments may have a form described below.
(2) A liquid material for medical device coating according to the above (1), which is an aqueous emulsion containing an aqueous solvent.
(3) A liquid material for medical device coating according to the above (1) or (2), wherein said first polysiloxane is polydimethylsiloxane having a silanol group at one end thereof and a trimethylsilyl group at the other end thereof.
(4) A liquid material for medical device coating according to any one of the above (1) through (3), wherein said second polysiloxane is polydimethylsiloxane having said silanol group at both ends thereof.
(5) A liquid material for medical device coating according to any one of the above (1) through (4), wherein said first polysiloxane has a molecular weight of 3,000 to 50,000.
(6) A liquid material for medical device coating according to any one of the above (1) through (5), wherein said second polysiloxane has a molecular weight of 200,000 to 400,000.
(7) A liquid material for medical device coating according to any one of the above (1) through (6), which contains a catalyst for promoting heat curing.
(8) A liquid material for medical device coating according to any one of the above (1) through (7), wherein a ratio between a content of said first polysiloxane contained in said liquid material for medical device coating and that of said second polysiloxane contained therein is 50:50 to 5:95.
(9) A liquid material for medical device coating according to any one of the above (1) through (8), wherein said polyfunctional silane is any one of nitrogen-containing silane, epoxy silane, and alkyl silane or a combination of not less than two kinds of said nitrogen-containing silane, said epoxy silane, and said alkyl silane.
(10) A liquid material for medical device coating according to any one of the above (1) through (9), wherein said polyfunctional silane has three functional groups.

The medical device having slidable coating layer of the present invention has a form described below.
(11) A medical device having slidable coating layer which contacts a medical member or is inserted into a living body when said medical device having slidable coating layer is used, wherein said medical device has a slidable coating layer formed at a portion thereof where said medical device contacts said medical member or an inside of said living body, wherein said slidable coating layer has a main structure containing a condensate of reactive polysiloxane having a silanol group at both ends thereof as a main component thereof and a large number of crosslinking parts formed of polyfunctional silane; and wherein said slidable coating layer contains polysiloxane bonded to at least one part of said crosslinking parts, and said polysiloxane has one end which is bonded to said crosslinking parts and other end which does not have a reactive functional group and is not bonded to said main structure nor to said crosslinking parts.

The above-described embodiments may have a form described below.
(12) A medical device having slidable coating layer according to the above (11), wherein said polysiloxane having said other end which does not have said reactive functional group and is not bonded to said main structure is bonded to 5 to 50% of said crosslinking parts of said slidable coating layer.
(13) A medical device having slidable coating layer according to the above (11) or (12), wherein said other end of said polysiloxane is a trimethylsilyl group.
(14) A medical device having slidable coating layer according to any one of the above (11) through (13), wherein said main structure contains said polysiloxane not having a reactive functional group and terminating at one end thereof.
(15) A medical device having slidable coating layer according to any one of the above (11) through (14), wherein said medical device is a gasket for a syringe; and said gasket for said syringe has said slidable coating layer formed at least at a portion thereof where said gasket contacts an inner surface of a barrel.

The syringe of the present invention has a form described below.
(16) A syringe having a barrel, a gasket for said syringe according to the above
(15) slidably accommodated inside said barrel, and a plunger which has been mounted on said gasket or can be mounted thereon.

The above-described embodiments may have a form described below.
(17) A syringe according to the above (16), wherein a liquid medicine is filled.
(18) A syringe according to the above (16) or (17), wherein said barrel is made of plastics.

## Claims

1. A liquid material for medical device coating for imparting slidability to said medical device,
wherein said liquid material for medical device coating contains first polysiloxane having a silanol group only at one end thereof, second polysiloxane having said silanol group at both ends thereof, and polyfunctional silane.

2. A liquid material for medical device coating according to claim 1, which is an aqueous emulsion containing an aqueous solvent.

3. A liquid material for medical device coating according to claim 1 or 2, wherein said first polysiloxane is polydimethylsiloxane having a silanol group at one end thereof and a trimethylsilyl group at the other end thereof.

4. A liquid material for medical device coating according to any one of claims 1 through 3, wherein said second polysiloxane is polydimethylsiloxane having said silanol group at both ends thereof.

5. A liquid material for medical device coating according to any one of claims 1 through 4, wherein said first polysiloxane has a molecular weight of 3,000 to 50,000.

6. A liquid material for medical device coating according to any one of claims 1 through 5, wherein said second polysiloxane has a molecular weight of 200,000 to 400,000.

7. A liquid material for medical device coating according to any one of claims 1 through 6, which contains a catalyst for promoting heat curing.

8. A liquid material for medical device coating according to any one of claims 1 through 7, wherein a ratio between a content of said first polysiloxane contained in said liquid material for medical device coating and that of said second polysiloxane contained therein is 50:50 to 5:95.

9. A liquid material for medical device coating according to any one of claims 1 through 8, wherein said polyfunctional silane is any one of nitrogen-containing silane, epoxy silane, and alkyl silane or a combination of not less than two kinds of said nitrogen-containing silane, said epoxy silane, and said alkyl silane.

10. A liquid material for medical device coating according to any one of claims 1 through 9, wherein said polyfunctional silane has three functional groups.

11. A medical device having slidable coating layer which contacts a medical member or is inserted into a living body when said medical device having slidable coating layer is used,
wherein said medical device has a slidable coating layer formed at a portion thereof where said medical device contacts said medical member or an inside of said living body,
wherein said slidable coating layer has a main structure containing a condensate of reactive polysiloxane having a silanol group at both ends thereof as a main component thereof and a large number of crosslinking parts formed of polyfunctional silane; and
wherein said slidable coating layer contains polysiloxane bonded to at least one part of said crosslinking parts, and said polysiloxane has one end which is bonded to said crosslinking parts and other end which does not have a reactive functional group and is not bonded to said main structure n or to said crosslinking parts.

12. A medical device having slidable coating layer according to claim 11, wherein said polysiloxane having said other end which does not have said reactive functional group and is not bonded to said main structure is bonded to 5 to 50% of said crosslinking parts of said slidable coating layer.

13. A medical device having slidable coating layer according to claim 11 or 12, wherein said other end of said polysiloxane is a trimethylsilyl group.

14. A medical device having slidable coating layer according to any one of claims 11 through 13, wherein said main structure contains said polysiloxane not having a reactive functional group and terminating at one end thereof.

15. A medical device having slidable coating layer according to any one of claims 11 through 14, wherein said medical device is a gasket for a syringe; and said gasket for said syringe has said slidable coating layer formed at least at a portion thereof where said gasket contacts an inner surface of a barrel.

16. A syringe having a barrel, a gasket for said syringe according to claim 15 slidably accommodated inside said barrel, and a plunger which has been mounted on said gasket or can be mounted thereon.

17. A syringe according to claim 16, wherein a liquid medicine is filled.

18. A syringe according to claim 16 or 17, wherein said barrel is made of plastics.
